# EUROPEAN PATENT APPLICATION

(11) **EP 4 170 011 A1**
(43) Date of publication of application: **26.04.2023**
(21) Application number: 21828862.9
(22) Date of filing: 23.06.2021
(51) Int. Cl.: C12M 1/42, G01N 27/416

(54) **METHOD FOR OXIDIZING OR REDUCING TARGET MOLECULE AND DEVICE FOR OXIDATION OR REDUCTION OF TARGET MOLECULE**

(30) Priority: 23.06.2020 JP 2020108218
(71) Applicant: Panasonic Intellectual Property Management Co., Ltd., Osaka-shi, Osaka 540-6207 (JP)
(72) Inventor: WAYAMA, Fumiya, Osaka-shi, Osaka 540-6207 (JP); HATSUGAI, Noriyuki, Osaka-shi, Osaka 540-6207 (JP); OKUMURA, Yasuaki, Osaka-shi, Osaka 540-6207 (JP)
(74) Representative: Novagraaf International SA
(86) International application number: PCT/JP2021/023751
(87) International publication number: WO 2021/261510

(57) **Abstract**

A target molecule redox method includes: a first process (S103) of bringing a liquid containing a target molecule to a non-flowing state, and causing electron transfer between an electron carrier immobilized on an electrode connected to an external power supply outside the liquid and the target molecule to oxidize or reduce the target molecule; and a second process (S104) of bringing the liquid to a flowing state. The first process (S103) and the second process (S104) are sequentially repeated.

## Description

### [Technical Field]

The present disclosure relates to the electrochemical redox of electron carriers.

### [Background Art]

Electron donors, which are electron carriers in reduced form, reduce electron acceptors by donating electrons to them. When an electron donor donates its electrons, it becomes oxidized and is thus unable to reduce a new target molecule (i.e., an electron acceptor). In in vivo redox reactions, however, since multiple substances function as electron donors and electron acceptors, even if a reduced electron carrier is oxidized, it can be reduced once again due to electron transfer between these substances. Since such a mechanism does not exist in in vitro redox reactions, oxidized electron carriers are typically reduced by using, for example, an electrochemical measuring device to donate electrons from an electrode.

### [Summary of Invention]

### [Technical Problem]

The present disclosure has an object to provide a target molecule redox method and a target molecule redox device that can efficiently oxidize or reduce target molecules throughout the entire reaction system.

### [Solution to Problem]

A target molecule redox method according to one aspect of the present disclosure includes: a first process of bringing a liquid containing an inactive target molecule to a non-flowing state, and causing electron transfer between an electron carrier immobilized on an electrode and the inactive target molecule to oxidize or reduce the inactive target molecule, the electrode being connected to an external power supply outside the liquid; and a second process of bringing the liquid to a flowing state. The first process and the second process are sequentially repeated.

### [Advantageous Effects of Invention]

The present disclosure can provide a target molecule redox method and a target molecule redox device that can efficiently oxidize or reduce target molecules throughout the entire reaction system.

### [Brief Description of Drawings]

[FIG. 1]

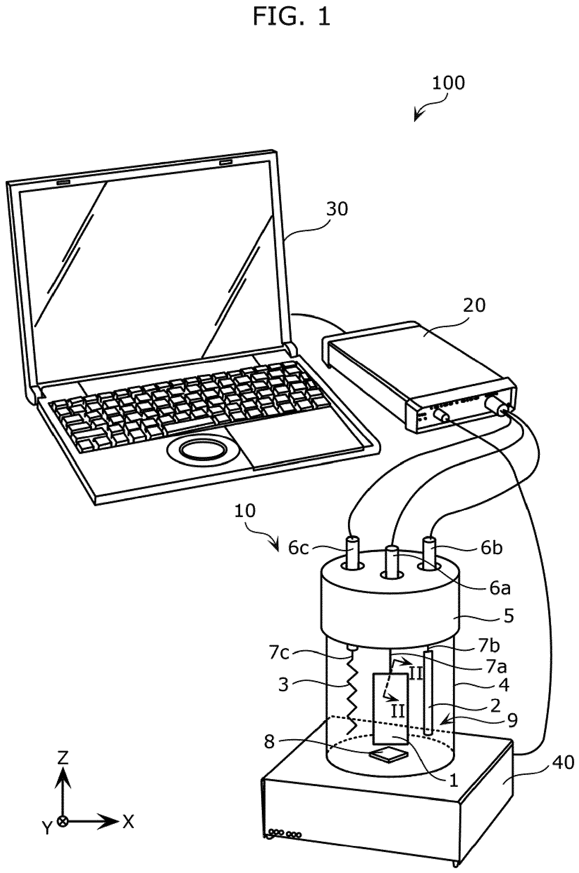

   FIG. 1 illustrates one example of the configuration of a target molecule redox device according to an embodiment of the present disclosure.
[FIG. 2]

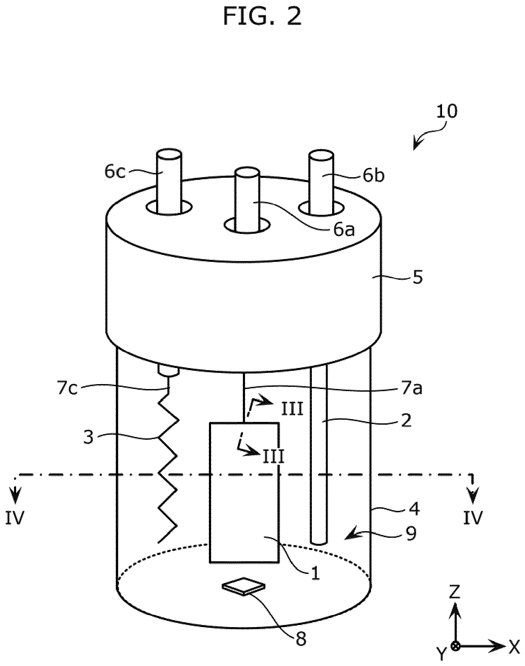

   FIG. 2 is a perspective view of a voltage applier according to an embodiment of the present disclosure.
[FIG. 3]

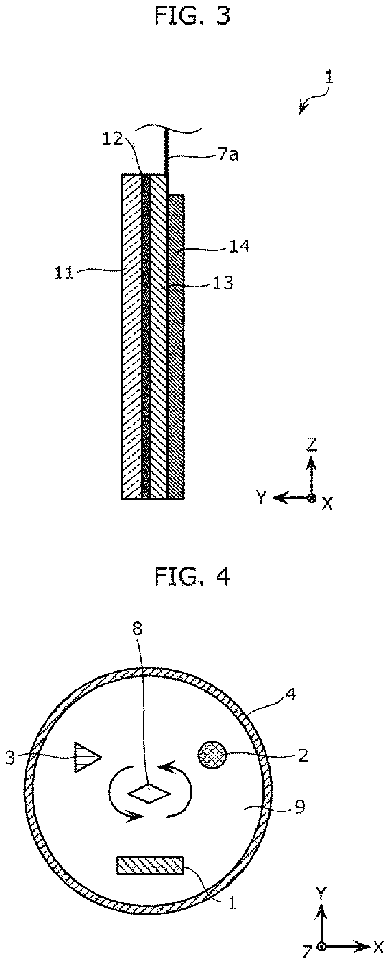

   FIG. 3 is a cross-sectional view taken at line III-III in FIG. 2.
[FIG. 4]
   FIG. 4 is a cross-sectional view taken at line IV-IV in FIG. 2.
[FIG. 5]

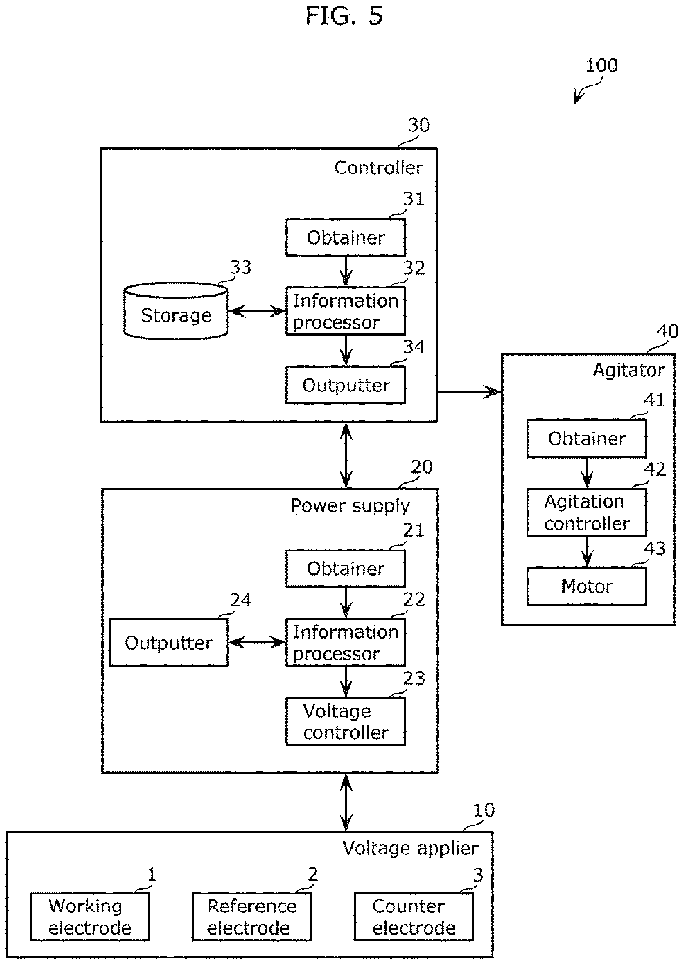

   FIG. 5 illustrates one example of the functional configuration of the target molecule redox device according to an embodiment of the present disclosure.
[FIG. 6]

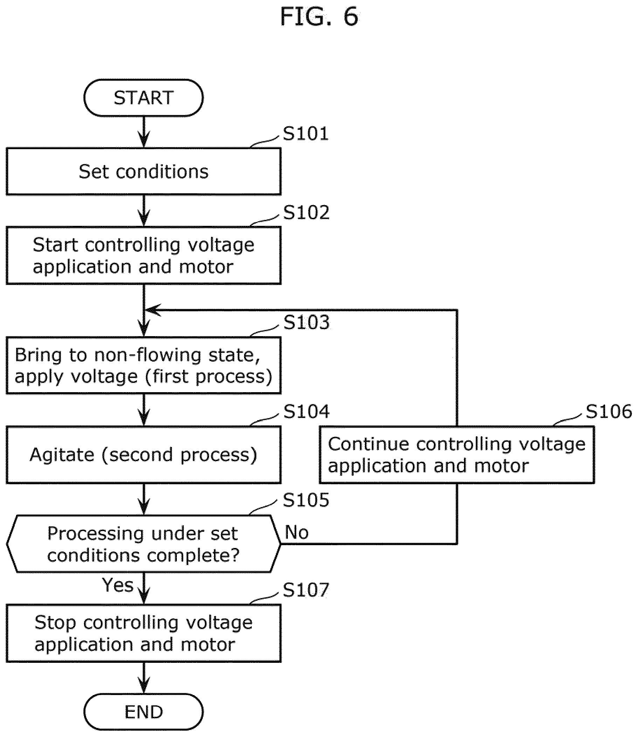

   FIG. 6 is a flowchart illustrating one example of the operation of the target molecule redox device according to an embodiment of the present disclosure.
[FIG. 7]

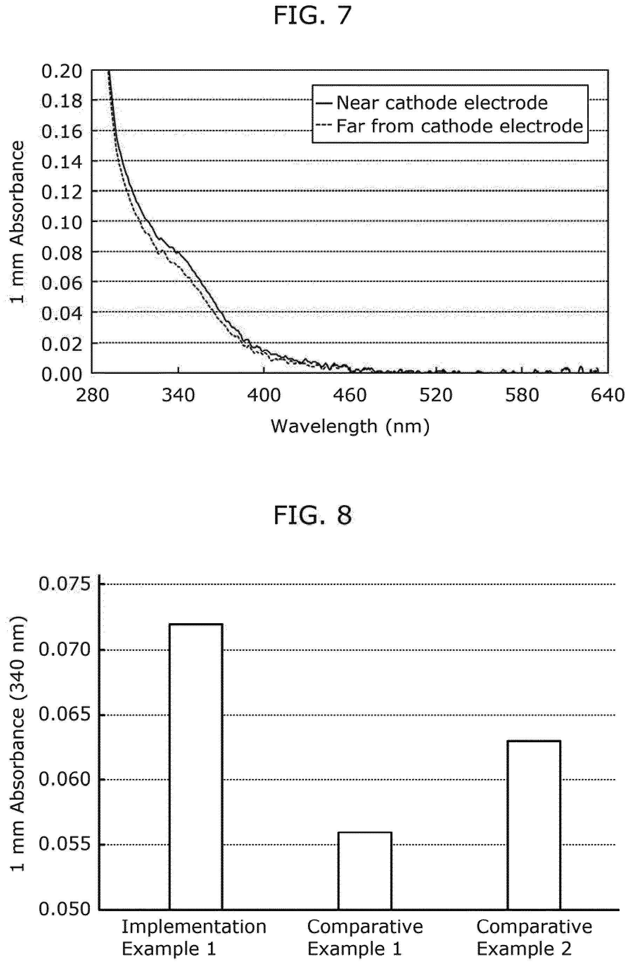

   FIG. 7 illustrates absorption spectra of the target molecule solution near the cathode electrode and near the counter electrode (i.e., far from the cathode electrode) for Implementation Example 1.
[FIG. 8]
   FIG. 8 is a graph illustrating absorbance at 340 nm of the entire target molecule solution for Implementation Example 1, Comparative Example 1, and Comparative Example 2.
[FIG. 9]

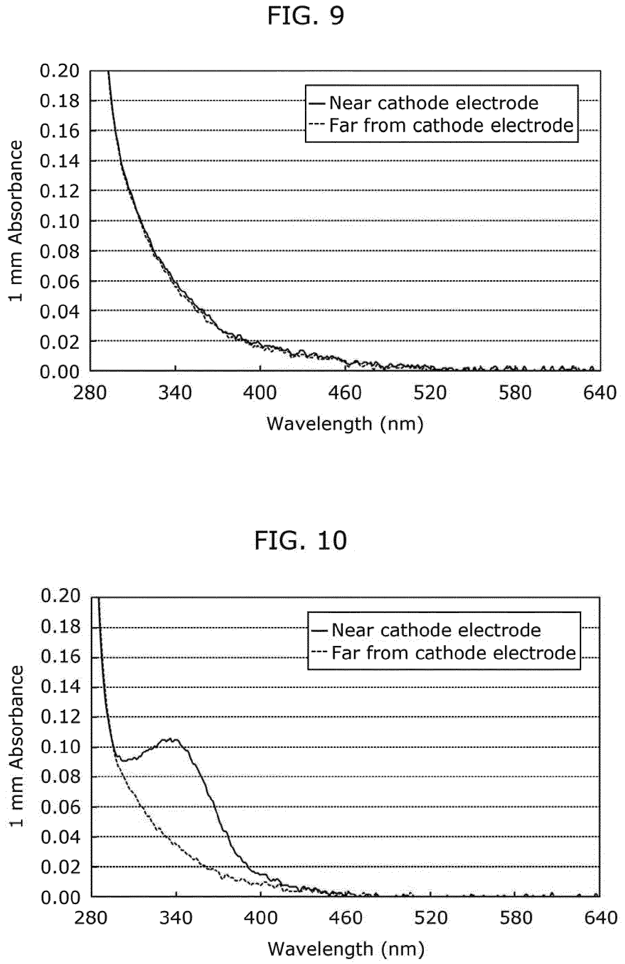

   FIG. 9 illustrates absorption spectra of the target molecule solution near the cathode electrode and near the counter electrode (i.e., far from the cathode electrode) for Comparative Example 1.
[FIG. 10]
   FIG. 10 illustrates absorption spectra of the target molecule solution near the cathode electrode and near the counter electrode (i.e., far from the cathode electrode) for Comparative Example 2.

### [Description of Embodiments]

### Underlying Knowledge Forming the Basis of the Present Disclosure

As described above, with the conventional technique, oxidized electron carriers are reduced by donating electrons to them from the cathode electrode of the electrochemical measuring device. However, it is difficult for the oxidized electron carriers to accept electrons directly from the cathode electrode. Therefore, by immobilizing electron donors onto the cathode electrode, the electron donors donate electrons accepted from the cathode electrode to the target molecules (i.e., the electron acceptors). In this way, the target molecules are reduced as a result of indirectly accepting electrons from the electrode.

Such an electron transfer reaction between the electron donors immobilized on the cathode electrode and the target molecules is a very localized reaction that occurs only around the cathode electrode; in the entire reaction system, many target molecules are not reduced (i.e., the activation efficiency is low with such a technique). Therefore, there is a desire to construct a reaction system capable of efficiently reducing target molecules throughout the entire reaction system.

In view of this, the present disclosure has an object to provide a target molecule redox method and a target molecule redox device that can efficiently oxidize or reduce the target molecule throughout the entire reaction system.

### One Aspect of the Present Disclosure

Hereinafter, one aspect of the present disclosure will be described.

A target molecule redox method according to one aspect of the present disclosure includes: a first process of bringing a liquid containing a target molecule to a non-flowing state, and causing electron transfer between an electron carrier immobilized on an electrode and the target molecule to oxidize or reduce the target molecule, the electrode being connected to an external power supply outside the liquid; and a second process of bringing the liquid to a flowing state. The first process and the second process are sequentially repeated.

This facilitates the formation of a stable reaction field because the electron transfer reaction between the electron carriers immobilized on the electrode and the target molecules takes place when the liquid is in a non-flowing state in the first process. This consequently improves the activation efficiency of target molecules. Moreover, by bringing the liquid to a flowing state in the second process, the activated target molecules are diffused from the vicinity of the electrode, whereby other target molecules in the liquid can easily move into the vicinity of the electrode. This increases the efficiency of the electron transfer reaction between the target molecules and the electron carriers since more target molecules in the liquid are able to undergo electron transfer reaction with the electron carriers. Moreover, since the first process and the second process are sequentially repeated, target molecules can be activated continuously. Therefore, according to this target molecule redox method, target molecules can be efficiently activated throughout the entire reaction system (i.e., the liquid).

In the target molecule redox method according to one aspect of the present disclosure, in the second process, the liquid may be agitated or shaken to bring the liquid to the flowing state.

This allows active target molecules to diffuse from the vicinity of the electrode and other target molecules in the liquid to move into the vicinity of the electrode, thus allowing the target molecules to be activated efficiently.

In the target molecule redox method according to one aspect of the present disclosure, a duration of the first process may be longer than a duration of the second process.

This allows for a longer time to activate more target molecules, thus allowing more target molecules to be activated.

In the target molecule redox method according to one aspect of the present disclosure, a ratio of the duration of the first process to the duration of the second process may be between 10:1 and 100:1, inclusive.

This allows sufficient time for the electron transfer reaction between electron carriers and target molecules, thus allowing more target molecules to be activated.

In the target molecule redox method according to one aspect of the present disclosure, the duration of the first process may be between 30 minutes and 90 minutes, inclusive, and the duration of the second process may be between 1 minute and 2 minutes, inclusive.

This allows sufficient time for the electron transfer reaction between electron carriers and target molecules, thus allowing more target molecules to be activated.

In the target molecule redox method according to one aspect of the present disclosure, the target molecule may be NADP⁺.

This produces NADPH as an active target substance, which can activate redox molecules, redox enzymes, and redox proteins involved in various redox reactions.

In the target molecule redox method according to one aspect of the present disclosure, the electrode may include a substrate including gold.

This allows for an electrode with low chemical reactivity and corrosion resistance. In addition, gold has high binding affinity for molecules with sulfur, nitrogen, or oxygen atoms, making surface modification easy, thus allowing for an electrode with added functionality.

In the target molecule redox method according to one aspect of the present disclosure, the electron carrier may be a 4,4'-bipyridinium derivative. For example, the electron carrier may be 1-methyl-1'-hexyl-4,4'-bipyridinium.

Thus, by using a 4,4'-bipyridinium derivative as the electron carrier, the electron carriers have a higher affinity for the enzyme. The electron carriers are therefore more likely to interact with, for example, coenzymes (NADP⁺) involved in redox reactions, as well as enzymes and proteins. Furthermore, the use of 1-methyl-1'-hexyl-4,4'-bipyridinium as the electron carrier facilitates immobilization of the electron carrier to the substrate.

In the target molecule redox method according to one aspect of the present disclosure, voltage may be applied via the external power supply in the first process.

This allows an efficient electron transfer reaction between electron carriers and target molecules, since electrons are donated from the electrode to the electron carriers.

A target molecule redox device according to one aspect of the present disclosure includes: an agitator that agitates a liquid containing a target molecule to bring the liquid to a flowing state; an electrode on which an electron carrier that oxidizes or reduces the target molecule by electron transfer with the target molecule is immobilized; a power supply that applies voltage to the electrode; and a controller that controls the power supply and the agitator. The controller switches the liquid between the flowing state and a non-flowing state by repeatedly causing the agitator to agitate and stop agitating.

This facilitates the formation of a stable reaction field because the electron transfer reaction between the electron carriers immobilized on the electrode and the target molecules takes place when the liquid is in a non-flowing state. This consequently improves the activation efficiency of target molecules. By performing control that switches the liquid between a flowing state and a non-flowing state, the activated target molecules are diffused from the vicinity of the electrode, whereby other target molecules in the liquid can easily move into the vicinity of the electrode. This increases the efficiency of the electron transfer reaction between the target molecules and the electron carriers since more target molecules in the liquid are able to undergo electron transfer reaction with the electron carriers. Furthermore, the activation and diffusion of target molecules can be performed repeatedly by switching the liquid between a flowing state and a non-flowing state, enabling continuous activation of target molecules. Therefore, according to this target molecule redox device, target molecules can be efficiently activated throughout the entire reaction system (i.e., the liquid).

In the target molecule redox device according to one aspect of the present disclosure, for example, the target molecule may be NADP⁺.

This produces NADPH as an active target substance, which can activate redox molecules, redox enzymes, and redox proteins involved in various redox reactions.

In the target molecule redox device according to one aspect of the present disclosure, for example, the electrode may include a substrate including gold.

This allows for an electrode with low chemical reactivity and corrosion resistance. In addition, gold has high binding affinity for molecules with sulfur, nitrogen, or oxygen atoms, making surface modification easy, thus allowing for an electrode with added functionality.

In the target molecule redox device according to one aspect of the present disclosure, for example, the electron carrier may be a 4,4'-bipyridinium derivative. For example, the electron carrier may be 1-methyl-1'-hexyl-4,4'-bipyridinium.

Thus, by using a 4,4'-bipyridinium derivative as the electron carrier, the electron carriers have a higher affinity for the enzyme. The electron carriers are therefore more likely to interact with, for example, coenzymes (for example, NADP⁺) involved in redox reactions, as well as enzymes and proteins. Furthermore, the use of 1-methyl-1'-hexyl-4,4'-bipyridinium as the electron carrier facilitates immobilization of the electron carrier to the substrate.

General or specific aspects of the present disclosure may be realized as a system, a method, a device, an integrated circuit, a computer program, a computer readable medium such as a CD-ROM, or any given combination thereof.

Hereinafter, embodiments will be described in detail with reference to the drawings.

Each of the embodiments described below shows a general or specific example of the present disclosure. The numerical values, shapes, materials, elements, the arrangement and connection of the elements, steps, the order of the steps, etc., indicated in the following embodiments are mere examples, and therefore do not intend to limit the claims. Therefore, among elements in the following embodiments, those not recited in any of the broadest, independent claims are described as optional elements. The figures are schematic illustrations and are not necessarily precise depictions. Elements that are essentially the same have the same reference signs in the figures, and duplicate description may be omitted or simplified.

The mutually orthogonal X-axis, Y-axis, and Z-axis directions illustrated in the figures will be used as appropriate in the description. In particular, the positive side in the Z-axis direction may be described as the upper side, and the negative side in the Z-axis direction may be described as the lower side.

In the present disclosure, terms indicating relationships between elements such as "parallel" and "perpendicular", terms indicating shapes of elements such as "rectangular", and numerical ranges refer not only to their strict meanings, but encompass a range of essentially equivalents, such as a range of deviations of a few percent.

In the figures of the present disclosure, dashed lines indicate the boundaries of what is not visible from the surface, as well as regions.

### [Embodiment]

Hereinafter, an embodiment of the present disclosure will be described in detail with reference to FIG. 1 through FIG. 5.

### Target Molecule Redox Device

### 1. Overview

First, an overview of the target molecule redox device according to an embodiment of the present disclosure will described with reference to FIG. 1. FIG. 1 illustrates one example of the configuration of target molecule redox device 100 according to an embodiment of the present disclosure.

Target molecule redox device 100 applies voltage to an electrode while a liquid containing target molecules is in a non-flowing state, oxidizes or reduces the target molecules by electron transfer between electron carriers, which are immobilized on the electrode, and the target molecules. The active target molecules are then diffused into the liquid by switching the liquid from a non-flowing state to a flowing state. By repeatedly switching the flow state of the liquid in this manner, the target molecules can be efficiently activated throughout the entire liquid.

As used herein, a non-flowing state of a liquid means, for example, that the liquid is not agitated or shaken (i.e., not subjected to external forces such as shearing forces or vibrations) and that no fluctuation or other motion is observed on the liquid surface.

### 2. Configuration

Next, the configuration of target molecule redox device 100 according to an embodiment of the present disclosure will be described with reference to FIG. 1 through FIG. 3.

Target molecule redox device 100 according to an embodiment of the present disclosure includes agitator 40 that agitates the liquid containing target molecules to bring the liquid to a flowing state, an electrode (cathode electrode 1) on which electron carriers that oxidize or reduce the target molecules by electron transfer with the target molecules are immobilized, power supply 20 that applies voltage to the electrode, and controller 30 that controls power supply 20 and agitator 40. The electrode on which the electron carriers are immobilized (hereafter referred to as cathode electrode 1) is a component of voltage applier 10.

### Voltage Applier

First, voltage applier 10 will be described with reference to FIG. 2. FIG. 2 is a perspective view of voltage applier 10 according to an embodiment of the present disclosure. Voltage applier 10 transfers electrons to and from the target molecules via the electron carriers immobilized on the electrode (cathode electrode 1). The electron transfer between the electron carriers and the target molecules causes the target molecules to be oxidized or reduced.

Voltage applier 10 is, for example, a three-electrode cell that includes cathode electrode 1 (also called a working electrode), reference electrode 2, counter electrode 3, cell 4, lid 5, terminals 6a, 6b, and 6c, and leads 7a, 7b, and 7c. Voltage applier 10 may be a two-electrode cell that includes, for example, a working electrode (cathode electrode 1) and counter electrode 3.

Next, cathode electrode 1 will be described with reference to FIG. 3. FIG. 3 is a cross-sectional view taken at line III-III in FIG. 2.

Cathode electrode 1 includes glass substrate 11, titanium deposition layer 12 deposited on glass substrate 11, cathode substrate 13 formed on titanium deposition layer 12, and reaction layer 14 including the electron carriers immobilized on cathode substrate 13.

Cathode electrode 1 is prepared by immobilizing, onto cathode substrate 13, a low molecular weight compound or enzyme capable of activating deactivated target molecules (i.e., inactive target molecules). This low molecular weight compound or enzyme functions as the electron carrier (also called an electron mediator). Cathode substrate 13 may be a conductive substrate made of conductive material. The conductive material may be, for example, a carbon material, a conductive polymer material, a semiconductor, or a metal. Carbon material examples include carbon nanotube, Ketjen black, glassy carbon, graphene, fullerene, carbon fiber, carbon fabric, and carbon aerogel. Conductive polymer material examples include polyaniline, polyacetylene, polypyrrole, poly(3,4-ethylenedioxythiophene), poly(p-phenylenevinylene), polythiophene, and poly(p-phenylene sulfide). Semiconductor examples include silicone, germanium, indium tin oxide (ITO), titanium oxide, copper oxide, and silver oxide. Metal examples include gold, platinum, silver, titanium, aluminum, tungsten, copper, iron, and palladium. The conductive material is not particularly limited as long as the conductive material is not decomposed by its own oxidation reaction. The thickness of cathode substrate 13 is not particularly limited.

The electron carrier immobilized on cathode substrate 13 is not particularly limited so long as it is a substance that enables electron transfer between the target molecule in the sample solution (also referred to as the liquid) and cathode substrate 13 (the conductive substrate described above). Electron carrier examples include a viologen compound, a bipyridine salt derivative, a quinone, and an indophenol. Viologen compound is the conventional name for N,N'-disubstituted-4,4'-bipyridinium with a substituent introduced on the two pyridine ring nitrogen atoms of 4,4'-bipyridine. The introduction of the substituent causes the ring nitrogen atom to become positively charged and function as an electron carrier. The bipyridinium salt derivative may have two chloride or bromide ions as counterions. The viologen compound is, for example, a 4,4'-bipyridinium derivative, such as 1,1'-dimethyl-4,4'-bipyridinium (methyl viologen), 1-methyl-1'-carboxylmethyl-4,4'-bipyridinium, 1,1'-dicarboxymethyl-4,4'-bipyridinium, 1-methyl-1'-aminoethyl-4,4'-bipyrid inium, 1,1'-diaminoethyl-4,4'-bipyridinium, 1-methyl-1'-ethyl-4,4'-bipyridinium, 1-methyl-1'-propyl-4,4'-bipyridinium, 1-methyl-1'-butyl-4,4'-bipyridinium, 1-methyl-1'-pentylhexyl-4,4'-bipyridinium, 1-methyl-1'-hexyl-4,4'-bipyridinium, 1-methyl-1'-heptyl-4,4'-bipyridinium, 1-methyl-1'-octyl-4,4'-bipyridinium, 1-methyl-1'-nonyl-4,4'-bipyridinium, or 1-methyl-1'-decyl-4,4'-bipyridinium, or a compound in which the methyl group at position 1 of these compounds is replaced by an ethyl group. Among these, the electron carrier may be 1-methyl-1'-hexyl-4,4'-bipyridinium.

Reference electrode 2 is an electrode that does not react with the components in sample solution 9 and maintains a constant potential, and is used to control the potential difference between cathode electrode 1 and reference electrode 2 to a constant level by power supply 20. Here, reference electrode 2 is a silver/silver chloride electrode. Counter electrode 3 is, for example, a platinum electrode.

Next, the arrangement of the three electrodes mentioned above will be described with reference to FIG. 4. FIG. 4 is a cross-sectional view taken at line IV-IV in FIG. 2. As illustrated in FIG. 4, cathode electrode 1, reference electrode 2, and counter electrode 3 are arranged in a cylindrical cell 4, surrounding the longitudinal center of cell 4. Agitating element 8 is located at the bottom of cell 4. The reaction layer including the electron carriers immobilized on cathode electrode 1 is arranged opposing the center of the vertical axis of the cell.

The electrochemical measurement according to an embodiment of the present disclosure includes cathode electrode 1, immobilized on the electrode surface of which is a low molecular weight compound and an enzyme as an electron carrier capable of reactivating a target molecule, an anode electrode as counter electrode 3, reference electrode 2, and agitating element 8 driven by controlling the flowing state and the non-flowing state of the sample solution to improve reaction efficiency. Cathode electrode 1 (the working electrode) may have a sufficiently larger surface area than the anode electrode (counter electrode 3).

Cell 4 is a holder for holding sample solution 9 in which inactive target molecules are present. Inside cell 4 is agitating element 8 that agitates sample solution 9. FIG. 1 and FIG. 2 illustrate an example in which cell 4 is cylindrical, but the shape of cell 4 is not limited to this example. Agitating element 8 will be described in greater detail later.

The inactive target molecule is, for example, NADP⁺. The inactive target molecule may be NAD⁺ or inactive ferredoxin.

Referring again to FIG. 1 and FIG. 2, terminal 6a, terminal 6b, and terminal 6c that electrically connect cathode electrode 1, reference electrode 2, and counter electrode 3 to power supply 20, respectively, are arranged on lid 5. Leads extend from each terminal, connecting the terminals to a battery. Although the lead of reference electrode 2 is not illustrated in FIG. 1 or FIG. 2, the lead extends from terminal 6b to reference electrode 2, and reference electrode 2 is connected to terminal 6b via this lead (not illustrated). Cathode electrode 1 is connected to terminal 6a via lead 7a, and counter electrode 3 is connected to terminal 6c via lead 7c.

### Power Supply

Next, power supply 20 will be described with reference to FIG. 5. FIG. 5 illustrates one example of the functional configuration of target molecule redox device 100 according to an embodiment of the present disclosure.

Power supply 20 applies voltage to an electrode (cathode electrode 1). More specifically, power supply 20 applies voltage between cathode electrode 1 and counter electrode 3 of voltage applier 10 and controls the potential between cathode electrode 1 and reference electrode 2 to a predetermined value in accordance with a control signal output from controller 30.

As illustrated in FIG. 5, power supply 20 includes, for example, obtainer 21, information processor 22, voltage controller 23, and outputter 24.

Obtainer 21 obtains a control signal output from controller 30 and outputs the obtained control signal to information processor 22. Obtainer 21 may also obtain data such as the potential of each electrode in voltage applier 10 and the current value flowing in sample solution 9. In such cases, outputter 24 outputs the data obtained by obtainer 21 to controller 30. The processing of such data in controller 30 will be described later.

Information processor 22 processes the information obtained by obtainer 21. For example, when information processor 22 obtains a control signal from obtainer 21, information processor 22 outputs the obtained control signal to voltage controller 23. When voltage controller 23 starts applying voltage to the electrodes in voltage applier 10, information processor 22 obtains data such as the potential of each electrode in voltage applier 10 and the current value flowing in sample solution 9, which are obtained from obtainer 21, and derives the voltage to be applied to cathode electrode 1 based on the obtained data. Information processor 22 outputs, to voltage controller 23, a control signal that controls the voltage of cathode electrode 1 with the derived voltage.

Voltage controller 23 applies voltage to each electrode of voltage applier 10 based on the control signal output from information processor 22.

Although FIG. 1 illustrates an example where power supply 20 and controller 30 are separate units, power supply 20 may include controller 30.

### Agitator

Agitator 40 agitates the liquid containing inactive target molecules (in this case, sample solution 9) to bring the liquid to a flowing state. More specifically, agitator 40 controls the rotation speed and rotation time of agitating element 8 that are set in voltage applier 10 by controlling the operation of motor 43 in accordance with the control signal output from controller 30.

As illustrated in FIG. 5, agitator 40 includes, for example, obtainer 41, agitation controller 42, and motor 43.

Obtainer 41 obtains a control signal output from controller 30 and outputs the obtained control signal to agitation controller 42.

Agitation controller 42 processes the information obtained by obtainer 41. For example, when agitation controller 42 obtains a control signal from obtainer 41, agitation controller 42 derives control conditions for motor 43 based on the obtained control signal and controls the operation of motor 43. More specifically, agitation controller 42 controls the movement (i.e., the rotation speed and the rotation time) of agitating element 8 by controlling the rotation speed and rotation time of motor 43.

Although FIG. 1 illustrates an example in which agitator 40 and voltage applier 10 are separate units, agitator 40 may be integrated with voltage applier 10. In such cases, agitator 40 may be arranged, for example, in lid 5 of voltage applier 10, and agitating element 8 may be, for example, an agitating blade that is attachable to and detachable from lid 5.

### Controller

Controller 30 processes information for controlling the application of voltage by power supply 20 and the movement of motor 43 of agitator 40. Controller 30 is realized, for example, by a processor, a microcomputer, or dedicated circuitry. FIG. 1 illustrates an example where controller 30 is a computer.

Controller 30 includes, for example, obtainer 31, information processor 32, storage 33, and outputter 34.

For example, obtainer 31 obtains information related to instructions input by a user (hereinafter referred to as instruction information), as well as data such as the potential of each electrode in voltage applier 10 and the current value flowing in sample solution 9, and outputs the obtained instruction information and data to information processor 32.

Information processor 32 derives, for example, the conditions under which voltage is to be applied to each electrode of voltage applier 10 (also called voltage application conditions), and liquid flow conditions, such as the flow rate of sample solution 9 and the switching of the flow state of sample solution 9, based on the instruction information obtained by obtainer 31. The instruction information may be, for example, the type of target molecule, the amount of sample solution 9, the amount of time until completion of the process, or the time of completion.

Information processor 32 may, for example, derive the reduction rate of the target molecule in the sample solution based on the data obtained by obtainer 31, and change the voltage application conditions and the liquid flow conditions in accordance with the derived reduction rate. For example, among the voltage application conditions derived based on the instruction information, information processor 32 may change the voltage application time, and may change the voltage applied to each electrode. For example, among the liquid flow conditions derived based on the instruction information, information processor 32 may change the timing of switching the flow state of the sample solution, and may change the flow rate. This allows controller 30 to re-derive (i.e., change) the voltage application conditions and liquid flow conditions derived based on the instruction information in accordance with the reduction state (reduction rate) of the target molecules, thus enabling more efficient reduction of the target molecules in the sample solution.

In addition, information processor 32 may derive a control signal to control the application of voltage by power supply 20 under the voltage application conditions, and may derive a control signal to control the operation of motor 43 under the liquid flow conditions. The voltage application conditions and the liquid flow conditions may be derived based on the instruction information or data obtained by obtainer 31, as described above, and, alternatively, may be set in advance by the user. Information processor 32 outputs these control signals to outputter 34.

Outputter 34 obtains the control signals derived by information processor 32 and outputs the control signals to power supply 20 and agitator 40.

Storage 33 stores data obtained by obtainer 31 and computer programs (for example, an application program for controlling power supply 20) executed by controller 30.

### 3. Operation

Next, the operation of target molecule redox device 100 according to an embodiment of the present disclosure will be described with reference to FIG. 6, together with FIG. 1 through FIG. 5. FIG. 6 is a flowchart illustrating one example of the operation of target molecule redox device 100 according to an embodiment of the present disclosure.

Although not illustrated in the figures, first, the preparation process performed before operating target molecule redox device 100 will be described. For example, the preparation process may be performed by the user. In the preparation process, first, sample solution 9 is prepared. The user introduces sample solution 9 containing inactive target molecules into cell 4 of voltage applier 10.

Next, the user inserts the electrodes into sample solution 9 and sets the electrodes in place. The electrodes are specifically cathode electrode 1, reference electrode 2, and counter electrode 3. Cathode electrode 1 is connected to lead 7a extending from terminal 6a arranged on lid 5, reference electrode 2 is connected to lead 7b extending from terminal 6b arranged on lid 5, and counter electrode 3 is connected to lead 7c extending from terminal 6c arranged on lid 5.

Next, the user inputs information related to instructions to target molecule redox device 100a, such as information regarding the type of target molecule, the amount of sample solution, the amount of time until completion of the process, and the time of completion.

In the above preparation process, the user introduced sample solution 9 containing the target molecules into cell 4, but target molecule redox device 100 may introduce sample solution 9 into cell 4. Stated differently, the above preparation process is exemplified as process performed by the user, but the preparation process may be performed by target molecule redox device 100. In such cases, target molecule redox device 100 may further include an introducer (not illustrated in the drawings), a collector (not illustrated in the drawings), an introduction port (not illustrated in the drawings), and an outlet port (not illustrated in the drawings). For example, the introducer may introduce sample solution 9 containing the inactive target molecules into cell 4 through an introduction port in cell 4. For example, the collector may reduce the inactive target molecules and collect sample solution 9 containing the active target molecules out of cell 4 through an outlet port in cell 4.

Next, operation of target molecule redox device 100 will be described. After the instruction information is input by the user, controller 30 sets the conditions for applying voltage to each electrode of voltage applier 10 and sets the liquid flow conditions (step S101). In setting the conditions, controller 30 derives the voltage application conditions and the liquid flow conditions based on the input instruction information. Controller 30 then outputs a control signal to power supply 20 that controls the application of voltage by power supply 20 under the derived voltage application conditions. Controller 30 outputs a control signal to agitator 40 that controls the operation of agitator 40 under the derived liquid flow conditions. In step S101, the user may select a program number associated with a combination of voltage application conditions and liquid flow conditions, and controller 30 may obtain the program number and set the voltage application conditions and liquid flow conditions.

Next, power supply 20 and agitator 40 obtain the control signals output from controller 30 and start controlling voltage application to the electrodes and motor 43, respectively, according to the respective control signals (step S102). Controller 20 starts applying voltage and agitator 40 stops motor 43 (i.e., brings sample solution 9 to a non-flowing state) (step S103). In step S103, for example, power supply 20 applies voltage between cathode electrode 1 and counter electrode 3 of voltage applier 10 and controls the potential between cathode electrode 1 and reference electrode 2 to a predetermined value. The predetermined value may be determined by the combination of the electron carrier and the target molecule used. At this time, agitator 40 does not operate motor 43 or rotates motor 43 at a speed that does not generate fluctuations in the liquid surface. As a result, inactive target molecules in sample solution 9 are reduced to active target molecules by accepting electrons via electron carriers immobilized on cathode electrode 1 while sample solution 9 is in a non-flowing state. This step is also referred to as the first process.

Next, agitator 40 rotates motor 43 at a predetermined speed to agitate sample solution 9 using agitating element 8 (step S104). This diffuses the target molecules activated in step S103 (i.e., the active target molecules) into sample solution 9, and inactive target molecules in sample solution 9 move to the vicinity of cathode electrode 1. In this step, agitator 40 switches from stopping agitation to agitating based on the liquid flow conditions so as to switch sample solution 9 from a non-flowing state to a flowing state. At this time, agitator 40 controls the rotational operation of motor 43 so as to control the speed and duration of rotation of agitating element 8. As a result, sample solution 9 in cell 4 is agitated and brought into a flowing state, and the active target molecules in the vicinity of cathode electrode 1 are diffused into sample solution 9. This makes it easier for inactive target molecules in sample solution 9 to move to the vicinity of cathode electrode 1. In step S104, power supply 20 may be controlled so as to stop applying voltage, and, alternatively, may be controlled so as to continue applying voltage.

Power supply 20 and agitator 40 sequentially repeat steps S103 and S104 (not illustrated in the drawings).

For example, the duration of step S103 may be longer than the duration of step S104, and their ratio may be between 10:1 and 100:1, inclusive. More specifically, the duration of step S103 is between 30 minutes and 90 minutes, inclusive, and the duration of step S104 is between 1 minute and 2 minutes, inclusive. As a result, more inactive target molecules are reduced to active target molecules in step S103, the agitation diffuses the active target molecules in the vicinity of cathode electrode 1 into sample solution 9, and new inactive target molecules move to the vicinity of cathode electrode 1. Repeating these processes increases the activation efficiency (i.e., reduction efficiency) of inactive target molecules throughout sample solution 9.

Next, controller 30 determines whether processing under the set conditions is complete (step S105). The conditions set are, for example, the duration (time) of the voltage application, the number of times the voltage is applied (for example, pulsed voltage), or the number of times sample solution 9 is switched between flow states. If controller 30 determines that the processing under the set conditions is not complete (No in step S105), controller 30 causes power supply 20 to continue applying voltage and agitator 40 to continue operating based on the liquid flow conditions (also referred to simply as operating) (step S106). Steps S103 and S104 are then repeated until the next decision (step S105) is made.

However, if controller 30 determines that processing under the set conditions is complete (Yes in step S105), controller 30 causes power supply 20 to end the application of voltage and agitator 40 to stop operating (step S107). This completes the activation of the inactive target molecules in sample solution 9.

### Implementation Examples

Hereinafter, implementation examples of the redox method of the target substance according to the present disclosure will be described in detail, but the following implementation examples are nothing more than examples, and the present disclosure is not limited to the following implementation examples in any way.

In the following implementation examples and comparative examples, oxidized nicotinamide adenine dinucleotide phosphate (NADP⁺) was used as the inactive target molecule (hereinafter referred to simply as target molecule).

### Implementation Example 1

### Target Molecule Solution Preparation

The target molecule solution was prepared to 1.0 mmol/L by dissolving NADP⁺ in phosphate-buffered saline (PBS) at pH 7.4.

### Cathode Electrode Preparation

A gold substrate was prepared by depositing titanium and gold on a glass substrate in the listed order. Next, 4-mercaptopyridine modified gold substrates were prepared by modifying 4-mercaptopyridine on the prepared gold substrates. Then, 1-methyl-1'-hexyl-4,4' - bipyridinium was immobilized onto the 4-mercaptopyridine monolayer on the gold substrate surface to prepare cathode electrode 1. Note that 1-methyl-1'-hexyl-4,4'-bipyridinium is an electron carrier that donates electrons to NADP⁺ thereby reducing it to NADPH (in other words, it reactivates NADP⁺).

### Application of Voltage to Target Molecule Solution

The target molecule solution (1.0 mM NADP⁺-PBS solution) was introduced into cell 4 of voltage applier 10 illustrated in FIG. 1, and the electrodes were set. A three-electrode system with the prepared cathode electrode 1 as the working electrode, a Pt (platinum) electrode as the counter electrode, and an Ag/AgCl (silver/silver chloride) electrode as the reference electrode was used. Next, an agitating element was placed in the target molecule solution, and voltage was applied to the target molecule solution while the agitating element was rotated at a predetermined rotational speed (rpm). More specifically, the target molecule solution was switched between a non-flowing state and a flowing state by controlling the movement of the agitating element through two types of rotational control, stopping rotation and rotating, while applying a predetermined voltage to the target molecule solution. This regulated the switching of the target molecule solution between a non-flowing state and a flowing state. The duration of application of voltage to the target molecule solution in the non-flowing state was sufficiently longer than the duration of application of voltage in the flowing state. These two types of control were sequentially repeated while the predetermined voltage was continuously applied to the target molecule solution.

### Confirming Reactivation of Target Molecules

After the application of voltage to the target molecule solution was completed, the target molecule solution was sampled from around the working electrode (the cathode electrode), the counter electrode, and the reference electrode. The absorbance of the sampled target molecule solution in the wavelength range including 340 nm (hereinafter simply referred to as absorbance) was then measured. Measurements were taken using a 1 mm cell. 340 nm is the absorption wavelength specific to NADPH.

The absorbance of the target molecule solution sampled around the working electrode (the cathode electrode) and the absorbance of the target molecule solution sampled around the counter electrode, which is located farthest from the working electrode, are illustrated in FIG. 7. As illustrated in FIG. 7, both the target molecule solution around the working electrode (near the cathode electrode in the figures) and the target molecule solution around the counter electrode (far from the cathode electrode in the figures) showed an absorption peak at 340 nm. The absorbance of the target molecule solution was 0.08 around the working electrode and 0.07 around the counter electrode, which is the electrode farthest from the working electrode. This confirmed that the target molecules, NADP⁺, were reduced to produce the active form, NADPH, throughout the target molecule solution.

The average absorbance at 340 nm of the target molecule solutions around the three electrodes was calculated as the absorbance of the entire target molecule solution. The calculation results are illustrated in FIG. 8. As illustrated in FIG. 8, in Implementation Example 1, the absorbance of the entire target molecule solution after voltage application was 0.072.

### Comparative Example 1

Comparative Example 1 was performed under the same conditions as Implementation Example 1, except that the agitating element was constantly rotated (i.e., the target molecule solution was always in a flowing state).

The absorbance of the target molecule solution sampled around the working electrode (the cathode electrode) and the absorbance of the target molecule solution sampled around the counter electrode, which is located farthest from the working electrode, are illustrated in FIG. 9. As illustrated in FIG. 9, both the target molecule solution around the working electrode (near the cathode electrode in the figures) and the target molecule solution around the counter electrode (far from the cathode electrode in the figures) did not show an absorption peak at 340 nm. This confirmed that the target molecules, NADP⁺, were not reduced throughout the target molecule solution.

The average absorbance of the target molecule solutions around the three electrodes was calculated as the absorbance of the entire target molecule solution. The calculation results are illustrated in FIG. 8. As illustrated in FIG. 8, in Comparative Example 1, the absorbance of the entire target molecule solution after voltage application was 0.056.

### Comparative Example 2

Comparative Example 2 was performed under the same conditions as Implementation Example 1, except that the agitating element was not rotated.

The absorbance of the target molecule solution sampled around the working electrode (the cathode electrode) and the absorbance of the target molecule solution sampled around the counter electrode, which is located farthest from the working electrode, are illustrated in FIG. 10. As illustrated in FIG. 10, although the target molecule solution around the working electrode (near the cathode electrode in the figures) showed an absorption peak at 340 nm, the target molecule solution around the counter electrode (far from the cathode electrode in the figures) did not show an absorption peak at 340 nm. This confirmed that the target molecules, NADP⁺, were reduced to produce the active form, NADPH, around the working electrode.

The average absorbance at 340 nm of the target molecule solutions around the three electrodes was calculated as the absorbance of the entire target molecule solution. The calculation results are illustrated in FIG. 8. As illustrated in FIG. 8, in Comparative Example 2, the absorbance of the entire target molecule solution after voltage application was 0.063.

### Observations

As illustrated in FIG. 8, Comparative Example 1 had a lower absorbance of the entire target molecule solution than both Implementation Example 1 and Comparative Example 2. When voltage is applied to the target molecule solution while the target molecule solution is agitated by rotating the agitating element (i.e., while the target molecule solution is in a flowing state), as in Comparative Example 1, the target molecules, NADP⁺, cannot stay around the working electrode (cathode electrode) long enough for the electron transfer reaction to occur. Therefore, an electron transfer reaction between the electron carriers immobilized on the working electrode and the target molecules is unlikely to occur, and the efficiency of electron donation from the electron carriers to the target molecules is considered to be poor.

As illustrated in FIG. 8, Comparative Example 2 had a higher absorbance of the entire target molecule solution than Comparative Example 1. When voltage is applied to the target molecule solution without rotating the agitating element (i.e., without agitating the target molecule solution, i.e., while the target molecule solution is in a non-flowing state), as in Comparative Example 2, the target molecules (NADP⁺) can remain around the working electrode long enough for the electron transfer reaction to occur. Therefore, the efficiency of electron donation from the electron carriers immobilized on the working electrode to the target molecules is considered to have improved. On the other hand, the absorbance around the counter electrode far from the working electrode is similar to that in Comparative Example 1, indicating that the electron transfer reaction took place only around the working electrode. Therefore, some degree of agitating is considered necessary to improve reaction efficiency.

As illustrated in FIG. 8, Implementation Example 1 had the highest absorbance at 340 nm for the entire target molecule solution. In Implementation Example 1, two types of control—the stopping of rotation of the agitating element and the rotating of the agitating element-were alternately repeated at predetermined time intervals (duration of stoppage > duration of rotation). Therefore, it is considered that the electron transfer reaction between the electron carriers on the working electrode and the target molecules occurs while the agitating element is stopped (i.e., while the target molecule solution is in a non-flowing state), and the target molecules activated around the working electrode diffuse throughout the target molecule solution while the agitating element is rotating (i.e., while the target molecule solution is in a flowing state). This indicates that the electron transfer reaction between the electron carriers and the target molecules was efficient throughout the target molecule solution.

This confirms that making the duration of voltage application while the target molecule solution is in a non-flowing state is longer than the duration that the target molecule solution is in a flowing state improves the activation efficiency of the target molecule.

Although the target molecule redox method and the target molecule redox device according to the present disclosure have been described above based on embodiments, the present disclosure is not limited to these embodiments. Various modifications to the above embodiments that may be conceived by those skilled in the art, as well as embodiments resulting from arbitrary combinations of elements from different embodiments that do not depart from the essence of the present disclosure are included the present disclosure.

### [Industrial Applicability]

According to the present disclosure, electron carriers can be repeatedly activated, making it widely applicable in any field that utilizes electron transfer reaction by electron carriers.

### [Reference Signs List]

- 1: cathode electrode
- 2: reference electrode
- 3: counter electrode
- 4: cell
- 5: lid
- 6a, 6b, 6c: terminal
- 7a, 7b, 7c: lead
- 8: agitating element
- 9: sample solution
- 10: voltage applier
- 11: glass substrate
- 12: titanium deposition layer
- 13: cathode substrate
- 14: reaction layer
- 20: power supply
- 21: obtainer
- 22: information processor
- 23: voltage controller
- 24: outputter
- 30: controller
- 31: obtainer
- 32: information processor
- 33: storage
- 34: outputter
- 40: agitator
- 41: obtainer
- 42: agitation controller
- 43: motor
- 100: target molecule redox device

## Claims

1. A target molecule redox method comprising:
a first process of bringing a liquid containing a target molecule to a non-flowing state, and causing electron transfer between an electron carrier immobilized on an electrode and the target molecule to oxidize or reduce the target molecule, the electrode being connected to an external power supply outside the liquid; and
a second process of bringing the liquid to a flowing state, wherein
the first process and the second process are sequentially repeated.

2. The target molecule redox method according to claim 1, wherein
in the second process, the liquid is agitated or shaken to bring the liquid to the flowing state.

3. The target molecule redox method according to claim 1 or 2, wherein
a duration of the first process is longer than a duration of the second process.

4. The target molecule redox method according to claim 3, wherein
a ratio of the duration of the first process to the duration of the second process is between 10:1 and 100:1, inclusive.

5. The target molecule redox method according to claim 4, wherein
the duration of the first process is between 30 minutes and 90 minutes, inclusive, and
the duration of the second process is between 1 minute and 2 minutes, inclusive.

6. The target molecule redox method according to any one of claims 1 to 5, wherein
the target molecule is NADP⁺.

7. The target molecule redox method according to any one of claims 1 to 6, wherein
the electrode includes a substrate including gold.

8. The target molecule redox method according to any one of claims 1 to 7, wherein
the electron carrier is a 4,4'-bipyridinium derivative.

9. The target molecule redox method according to claim 8, wherein
the electron carrier is 1-methyl-1'-hexyl-4,4'-bipyridinium.

10. The target molecule redox method according to any one of claims 1 to 9, wherein
voltage is applied via the external power supply in the first process.

11. A target molecule redox device comprising:
an agitator that agitates a liquid containing a target molecule to bring the liquid to a flowing state;
an electrode on which an electron carrier that oxidizes or reduces the target molecule by electron transfer with the target molecule is immobilized;
a power supply that applies voltage to the electrode; and
a controller that controls the power supply and the agitator, wherein
the controller switches the liquid between the flowing state and a non-flowing state by repeatedly causing the agitator to agitate and stop agitating.

12. The target molecule redox device according to claim 11, wherein
the target molecule is NADP⁺.

13. The target molecule redox device according to claim 11 or 12, wherein
the electrode includes a substrate including gold.

14. The target molecule redox device according to any one of claims 11 to 13, wherein
the electron carrier is a 4,4'-bipyridinium derivative.

15. The target molecule redox device according to claim 14, wherein
the electron carrier is 1-methyl-1'-hexyl-4,4'-bipyridinium.
